# EUROPEAN PATENT APPLICATION

(11) **EP 4 206 335 A1**
(43) Date of publication of application: **05.07.2023**
(21) Application number: 22215734.9
(22) Date of filing: 22.12.2022
(51) Int. Cl.: C12Q 1/6886

(54) **METHOD FOR IDENTIFYING GASTRIC CANCER-ASSOCIATED MUTATIONS IN BLOOD USING A GENE PANEL**

(30) Priority: 30.12.2021 LT 2021577
(71) Applicant: Lietuvos sveikatos mokslu universitetas, 44307 Kaunas (LT)
(72) Inventor: Streleckiene, Greta, 44307 Kaunas (LT); Skieceviciene, Jurgita, 44307 Kaunas (LT); Kupcinskas, Juozas, 44307 Kaunas (LT)
(74) Representative: Zaboliene, Reda

(57) **Abstract**

A panel of 38 genes was created after whole-exome sequencing of 29 gastric cancer patients. Using this panel and performing next-generation sequencing, somatic mutations can be evaluated, and the results obtained can be used to predict the course of the disease in patients with various tumors assess relapse, and predict the disease.

## Description

### TECHNICAL FIELD

The subject matter described herein relates to the method of mutation detection in nucleic acids, and more particularly, to the detection of cancer-associated somatic mutations in cell-free DNA isolated from body fluids.

### BACKGROUND

Gastric cancer is one of the most common and lethal cancers in Lithuania and worldwide. Mortality from this disease is approaching the level of mortality from cardiovascular diseases. Personalized biomarker-based treatment of cancer patients is analyzed in many clinical trials increasingly. Therefore, in recent years gastric cancer research has focused on the development of new molecular diagnostic tools and technologies that would allow more accurate early diagnostics, monitor the course of the disease, and apply the principles of personalized medicine in gastric cancer treatment. Studies have shown that through the analysis of the cell-free DNA in the blood, it is possible to diagnose an oncological disease and monitor its progress.

US patent document US2020181711A1 describes a similar method for screening cancer, where the expression of EN2 and SATB2 genes in samples of patients' body fluids is used to diagnose and screen cancerous processes. This patent document only evaluates the expression of the two genes mentioned above and does not provide an optimized gene set that would allow a more accurate analysis of gastric cancer. US patent document US2021002727A1 describes a method to identify diseaseassociated gene variants by analyzing and comparing the whole-exome sequencing data of patients and healthy controls. Although the comparison of DNA sequences between patients and healthy controls may reveal more disease-related variants of DNA sequences, this method also generates more false-positive results due to interindividual variation.

The closest patent document, US2019256924A, describes a method for testing multiple oncological diseases and includes biomarker-based analysis of free circulating molecular material in blood samples of cancer patients. Genes involved in the screening panel are KRAS, TP53, CDKN2A, SMAD4, PIK3CA, HRAS, CDKN2A, AKT1, CTNNB1, APC, EGFR, GNAS, PPP2R1A, BRAF, FBXM7, PTEN and FGFR2. Although some of the genes used in the patent document overlap with the invention described herein, the gene panel of the invention US2019256924A is not optimized for gastric cancer.

### SUMMARY OF THE INVENTION

The developed panel of 38 genes covers a region of about 390 kilobases in the genome. The application of this panel by using next-generation sequencing analysis (the GATK Best Practices somatic short variants workflow (PMID: 25431634), which includes bioinformatics tools such as Burrows-Wheeler Aligner (BWA), Samtools, Mutect2, etc.) can be implemented for the evaluation of somatic DNA mutations associated with oncogenesis in patients' blood. Whole-exome sequencing was performed using the hybridization of specific primers to enrich the sequences of the coding part of the genome (commercial xGen Exome Research Panel v1, IDT kit). The obtained data can be applied for disease monitoring, prognosis, and relapse evaluation in patients with various cancers.

Gastric cancer patients (n=29) were recruited in LSMU Kaunas clinics Department of Gastroenterology and a whole-exome sequencing of the gastric mucosa biopsy samples was performed. Next, based on the whole-exome sequencing results, a panel of 38 genes was created. This panel and next-generation sequencing analysis can be applied for monitoring the oncogenesis by evaluating tumor-derived somatic mutations in a minimally invasive way - in the blood plasma of patients. Mutations that were identified include single nucleotide variants and small insertions or deletions. Recent research supports the idea that cell-free DNA analysis can be applied to disease progression monitoring, relapse assessment, and prognosis in patients with various tumors.

This cancer-associated mutation detection method is used to evaluate mutations present in cell-free DNA fragments isolated from a patient's biological samples using a panel of genes: ACVR2A, CDH1, ERBB4, GLI3, MSH6, PREX2, SYNE1, TRRAP, APC, CTNNB1, FAT1, KMT2C, MUC16, PTEN, SMAD4, ZIC4, ARID1A, EGFR, FAT4, KRAS, PBRM1, RHOA, SPEN, TTN, ATM, EPHB1, FBXW7, MACF1, PIK3CA, RIMS2, STK11, CCND1, ERBB2, FHIT, MLH1, PKHD1, RNF43 and TP53 and genesspecific primers. Mutations are evaluated by comparing the patients' sequencing data with the reference human genome.

The biological sample is usually a sample of body fluids, mostly a blood sample. Cell-free DNA isolated from body fluids, specifically blood plasma, is used for mutation analysis. A mutational profile of the gene panel of cell-free DNA is implemented for quantitative and qualitative analysis. Once deleterious single nucleotide variant, insertion, or deletion is identified, they are further implemented in quantitative and qualitative analysis to predict cancer. This gene panel is developed for the identification of gastric cancer-related mutations specifically.

### DETAILED DESCRIPTION OF THE INVENTION

For cell-free DNA extraction peripheral blood was collected using K₂EDTA tubes (one 10 mL tube for one patient; Becton, Dickinson and Company, Franklin Lakes, NJ) during a standard phlebotomy procedure. First, plasma is separated from erythrocytes within 2 hours from blood draw by using an optimized double centrifugation protocol (Streleckiene G, Forster M, Inciuraite R, Lukosevicius R, Skieceviciene J. Effects of Quantification Methods, Isolation Kits, Plasma Biobanking, and Hemolysis on Cell-Free DNA Analysis in Plasma. Biopreserv Biobank. 2019;17(6):553-561. PMID: 31343271). Purification of cell-free DNA from blood plasma is performed using a commercial isolation kit QIAamp Circulating Nucleic Acid isolation kit (Qiagen, Hilden, Germany). Next, a gene panel consisting of 38 genes and gene-specific primers is used for the enrichment of genes and next-generation sequencing is performed. Sequences generated are aligned to the reference human genome (GRCh37/hg19) and sequence processing is performed: removal of sequencing adapters, quality control, and collapsing of the sequences into "families" based on unique molecular identifiers (UMI). The identified genetic variants are later annotated and their impact on protein production is evaluated by using databases such as dbSNP/ ClinVar, COSMIC, etc. Only deleterious somatic variants (single nucleotide variants and insertions or deletions) are involved in the downstream analysis. Our study showed that qualitative (i.e. detectable somatic mutation in blood plasma) and quantitative (i.e. number of detectable mutations, frequency of mutated allele) analysis of cell-free circulating DNA in plasma has prognostic significance for patient survival and tumor spread.

**Table 1. Genes involved in gene panel**

| | | | | | | | |
|---|---|---|---|---|---|---|---|
| ACVR2A | CDH1 | ERBB4 | GLI3 | MSH6 | PREX2 | SYNE1 | TRRAP |
| APC | CTNNB1 | FAT1 | KMT2C | MUC16 | PTEN | SMAD4 | ZIC4 |
| ARID1A | EGFR | FAT4 | KRAS | PBRM1 | RHOA | SPEN | TTN |
| ATM | EPHB1 | FBXW7 | MACF1 | PIK3CA | RIMS2 | STK11 | |
| CCND1 | ERBB2 | FHIT | MLH1 | PKHD1 | RNF43 | TP53 | |

This approach allowed determining the somatic variants in plasma cell-free DNA samples for 21 of 23 patients (91.3 %) and tumor tissue matching alterations for 11 of 23 samples (47.8%). In comparison, previously reported data of other researchers' plasma cell-free DNA mutational concordance with tissue ranged from 33.9% to 58% (Fang W-L, et al, 2016; Kim YW, et al, 2019; Lan J, et al, 2020).

In addition, our data also showed that even relatively small gastric cancer tumors (10 % of T1-T2 status according to TNM classification) could shed detectable amounts of cell-free DNA into the bloodstream. This suggests that the identification of mutations can lead to early detection of the disease and thus improve patient survival.

By using the sequencing data of this gene panel, mutations were quantified and analyzed for association with patient survival. The strong association between the number of mutations and survival shows that the analysis of mutations in plasma cell-free DNA could be applied to the patient prognosis estimation and personalized treatment strategies when mutations are detected in genes associated with the response to therapy. This study showed that in patients with i) 1-2 cell-free DNA somatic sequence alterations, in genes involved in the gene panel, median survival time was 469 days; ii) 3-6 cell-free DNA somatic sequence alterations, in genes involved in the gene panel, median survival time was 315 days; iii) more than 6 cfDNA somatic sequence alterations, in genes involved in gene panel, was and 44 days. The median survival time for the patients without somatic sequence alterations was the longest and reached 803 days. Differences between these groups were statistically significant *P* = 0.008 (when the alfa probability criterion is 0.05). In addition, Cox proportional hazards model that included patients' demographics and tumor characteristics was used. Results showed a significant effect of more than 6 variants detected in plasma (P adjusted = 0.0186) for a prediction of a shorter lifespan.

## Claims

1. A method for detecting cancer-related mutations in sample, comprising:
by evaluating cancer-associated mutations using a gene panel consisting of: ACVR2A, CDH1, ERBB4, GLI3, MSH6, PREX2, SYNE1, TRRAP, APC, CTNNB1, FAT1, KMT2C, MUC16, PTEN, SMAD4, ZIC4, ARID1A, EGFR, FAT4, KRAS, PBRM1, RHOA, SPEN, TTN, ATM, EPHB1, FBXW7, MACF1, PIK3CA, RIMS2, STK11, CCND1, ERBB2, FHIT, MLH1, PKHD1, RNF43 and TP53 genes, and gene-specific primers, wherein said evaluation comprises targeted (gene) next-generation sequencing and comparison of the sequences with the reference human genome.

2. The method of claim 1, wherein the sample is a sample of body fluids.

3. The method of claim 2, wherein the body fluid sample is a blood sample.

4. The method according to any one of claims from 1 to 3, wherein gene panel DNA fragments used for somatic mutation analysis are obtained from cell-free DNA.

5. The method according to any one of claims from 1 to 4, wherein cell-free DNA is isolated from blood plasma.

6. The method according to any one of claims from 1 to 5, wherein the deleterious somatic mutations comprise single nucleotide substitutions and insertions or deletions.

7. The method according to any one of claims from 1 to 6, the detected mutations are cancer-associated somatic mutations.

8. The method of claim 7, wherein the cancer-associated mutations detected are gastric cancer-associated mutations.
